(19) Europäisches Patentamt
European Patent Office
Office européen des brevets

(11) Publication number: **0 236 848 A1**

# (12) EUROPEAN PATENT APPLICATION

(21) Application number: 87102662.1

(51) Int. Cl.[4]: C07C 43/225 , C08K 5/06

(22) Date of filing: 25.02.87

(30) Priority: 03.03.86 DE 3606829

(43) Date of publication of application: 16.09.87 Bulletin 87/38

(84) Designated Contracting States: BE DE FR GB NL

(71) Applicant: Chemische Fabrik Kalk GmbH
Kalker Hauptstrasse 22 Postfach 91 01 57
D-5000 Köln 91(DE)

(72) Inventor: Büttgens, Walter
Petersbergstrasse 2
D-5340 Bad Honnef 1(DE)
Inventor: Kerscher, Utto, Dr.
Am Beissel 25
D-5042 Erfstadt 12(DE)
Inventor: Neukirchen, Ernst
Vorsterstrasse 75
D-5000 Köln 91(DE)

(74) Representative: Werner, Hans-Karsten, Dr. et al
Deichmannhaus am Hauptbahnhof
D-5000 Köln 1(DE)

## (54) Bis- or poly-(bromo-4-methyl-phenoxy)-alkanes and -alkenes, process for preparing same and use thereof as uv-stable fireprotective agents for synthetic materials.

(57) Disclosed are novel bis-or poly-(bromo-4-methylphenoxy)-alkanes and -alkenes having the formula (1)

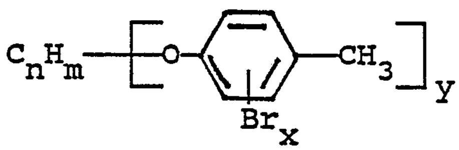

(1)

wherein
n is an integer of from 1 to 6,
m is 2 (n + z) -y,
x is an integer of from 1 to 4,
y is an integer of from 2 to 4 and
z is 0 or 1,
and a process for preparing said compounds by reacting alkali salts of nucleus-brominated p-cresols with bis-or poly-haloalkanes or -alkenes, preferably in a glycol monoalkylether as reaction medium. The materials are employed as UV-stable low-migrating fire-protective agents for thermoplastic synthetic materials, and more specifically for polybutylene terephthalate or acrylonitrile-butadiene-copolymer molding compositions, in combination with antimony trioxide.

EP 0 236 848 A1

## BIS-OR POLY-(BROMO-4-METHYLPHENOXY)-ALKANES AND -ALKENES, PROCESS FOR PREPARING SAME AND USE THEREOF AS UV-STABLE FIRE-PROTECTIVE AGENTS FOR SYNTHETIC MATERIALS

The present invention relates to bis-or poly-(bromo-4-methylphenoxy)alkanes and -alkenes having the formula (1)

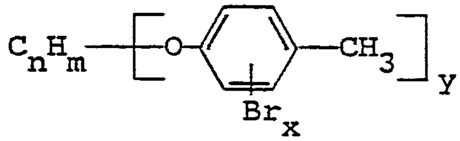

(1)

wherein
n is an integer of from 1 to 6,
m is 2 (n + z) -y,
x is an integer of from 1 to 4,
y is an integer of from 2 to 4 and
z is 0 or 1,
the preparation thereof and the use of these products as UV-stable fire-protective agents for synthetic materials, more specifically for polybutylene terephthalate or ABS molding compositions.

### BACKGROUND OF THE INVENTION

The preparation of bis(halo-4-methylphenoxy)-alkanes by the reaction of halogenated phenols with dihaloalkanes in a reaction medium in the presence of an alkali has been known from the chemical-technical literature. As reacttion media there have been mentioned acetone, lower alchohols or also aqueous systems. However, insufficient yields are obtained through the use of said reaction media. Tests conducted by Applicants to react tetra-bromo-p-cresol with dibromomethane in the presence of sodium hydroxide using acetone as a solvent gave only very poor yields. In further experiments carried out in ethanol, n-butanol or ethylene glycol, satisfactory yields likewise could not be obtained. The resulting products had to be recrystallized from an aromatic solvent such as, for example, toluene, xylene or dichlorobenzene in order to have the purity required to stand the thermal load exerted during the incorporation in thermoplastic synthetic materials without adversely affecting the properties of the synthetic materials.

Thus, it is a primary object to find a process for preparing bis-or poly-(bromo-4-methylphenoxy)-alkanes and -alkenes, whereby these products are obtained with high purity and in good yields so that they may be used as fire-protective agents for thermoplastic synthetic materials and the synthetic materials provided therewith do not exhibit changes in color under the action of UV irradiation.

### SUMMARY OF THE INVENTION

It has been found that said object can be attained by reacting an alkali salt of a nucleus-brominated p-cresol having the formula (2)

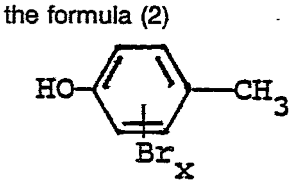

(2)

wherein x is an integer of from 1 to 4, with a bis-or poly-haloalkane or -alkene in a glycol ether, more specifically in a monoglycol ether such as, for example, 2-methoxyethanol or 1-methoxy-2-propanol. As the brominated alkali p-cresolates there may be employed the sodium salts as well as the potassium salts. As the halo-alkane and -alkene compounds, respectively, there may in particular be used substances having the general formula (3)

$C_nH_m Hal_y$ (3)

wherein
Hal represents chlorine, bromine or iodine,
n is an integer of from 1 to 6,
m is 2 (n + z) -y and

y is an integer of from 2 to 4.

DETAILED DESCRIPTION

The preferred haloalkanes and haloalkenes are dichloromethane, dibromomethane, dichloroethane, dibromoethane, dichloropropane, dibromopropane, tetrachloromethane, 1,1,1-trichloroethane or 1,1,2,2-tetrachloroethene, however still further staring material of this type may be employed.

The process according to the invention has made readily available a group of new substances having the formula (1) as described above.

These compounds of the formula (1) according to the invention can be obtained in good yields and in high purity. Typical compounds according to the invention are the following:
Bis(dibromo-4-methylphenoxy)-methane,
Bis(tetrabromo-4-methylphenoxy)-methane,
1,1-Bis(dibromo-4-methylphenoxy)-ethane,
1,1-Bis(tetrabromo-4-methylphenoxy)-ethane,
1,1-Bis(dibromo-4-methylphenoxy)-ethene,
1,1-Bis(tetrabromo-4-methylphenoxy)-ethene,
1,2-Bis(dibromo-4-methylphenoxy)-ethane,
1,2-Bis(tetrabromo-4-methylphenoxy)-ethane,
1,2-Bis(dibromo-4-methylphenoxy)-propane,
1,2-Bis(tetrabromo-4-methylphenoxy)-propane,
1,3-Bis(dibromo-4-methylphenoxy)-propane,
1,3-Bis(tetrabromo-4-methylphenoxy)-propane,
Tris(dibromo-4-methylphenoxy)-methane,
Tris(tetrabromo-4-methylphenoxy)-methane,
1,1,1-Tris(dibromo-4-methylphenoxy)-ethane,
1,1,1-Tris(tetrabromo-4-methylphenoxy)-ethane,
Tetrakis(dibromo-4-methylphenoxy)-methane,
Tetrakis(tetrabromo-4-methylphenoxy)-methane,
1,1,2,2-Tetrakis(dibromo-4-methylphenoxy)-ethene,
1,1,2,2-Tetrakis(tetrabromo-4-methylphenoxy)-ethene,

The products prepared in accordance with the present invention can actually be used without prior purification such as recrystallization as non-discolorizing fire-protective agents for thermoplasts, more specifically for polybutylene terephthalate and ABS molding compositions. Upon combination with antimony trioxide, molding compositions are obtained the mechanistic properties of which are virtually unchanged from those of untreated thermoplasts, while no discolorations occur even under the action of UV irradiation. Also no significant migration of the incorporated flame-protective agents is observed.

For carrying out the preparation process according to the invention, first an alkali bromo-p-cresolate is prepared in the reaction medium from bromo-p-cresol of the formula (2) and an alkali hydroxide. Upon heating at 70 °C a clear solution of the alkali salt is obtained which salt is subsequently reacted by the addition of a di-or poly-haloalkane or -alkene at a temperature of about 120 °C. Once the reaction has been completed and the reaction mixture has been allowed to cool, the precipitated reaction can be separated by filtration. After washing and drying it is obtained as a pure product with an approximately theoretical bromine content in a yield of about 93 to 97% of the theoretical yield.

The process according to the present invention is applicable in the case that in a preceding process step p-cresol has been reacted with, for example, bromine, to form bromo-p-cresol and in the same reaction vessel the prepared bromo-p-cresol without having been isolated is reacted in the manner according to the invention with di-or poly-halo-alkanes or -alkenes to give a bis-or poly-(bromo-4-methyl-phenoxy)alkane or -alkene.

To this end, first pure p-cresol is dissolved in an inert solvent such as, for example, dibromomethane, and then reacted with the amount of bromine required for the nucleus-substitution with the desired number of bromine atoms, if desired in the presence of a halogenation catalyst such as, for example, aluminum chloride. Upon completion of the reaction, the aluminum compound is removed from the reaction mixture by stirring with water and repeated washing steps. After separation of the aqueous phase, part of the glycol ether is added which is to be used according to the invention for the reaction of, for example, bromo-p-cresol with haloalkanes. From the organic mixture the reaction medium used for the bromination of p-cresol

is completely distilled off at a bottoms temperature of 120 °C. The bromo-p-cresol formed is diluted with further glycol ether and is reacted with halo-alkane or -alkene in the manner described above. Re-use of the filtered mother liquor will further increase the yield of the bis-or poly-(bromo-4-methylphenoxy)-alkanes or -alkenes without a deterioration in quality.

In order to produce fire-protected polybutylene or ABS molding compositions, the products according to the invention together with antimony trioxide are admixed with the respective synthetic material, extruded at a temperature of about 250 °C to 260 °C and granulated. Standard test specimens are prepared from the granules for carrying out the quality tests. Said tests showed a good fire-resistance of the synthetics test specimens, a high UV stability and no surface coating due to migrated fire-protective agent. Disadvantageous alterations of the mechanical and physical properties of the synthetic material are not effected by the incorporated products.

The process according to the invention and the use of the products according to the invention are further illustrated by the following examples.

EXAMPLE 1 (Preparation)

In a four-neck flask equipped with a reflux condenser on its top, a dropping funnel and a thermometer, 400 g of 1-methoxy-2-propanol (propylene glycol monomethylether) as reaction medium, 212 g of tetrabromo-p-cresol (0.5 moles) and 21 g of sodium hydroxide (0.525 moles) are mixed in this sequence. Upon heating at 70 °C a clear solution is formed, to which 65 g dibromomethane (0.374 moles) are added within 5 minutes. The reaction mixture is heated to 120 °C within a period of 1 hour and maintained at this temperature for another 11 hours. The mixture is allowed to cool, and the precipitated reaction product is filtered off by suction and washed with 50 g of 1-methoxy-2-propanol. After sucking-off well, the filter cake is suspended in water, stirred for 2 hours and filtered by suction, then once more washed with two liters of water and, after sucking-off until well dry, dried in a fresh-air drying oven at a temperature of 80 °C; 205 g of slightly beige-colored bis(tetrabromo-4-methyl-phenoxy)-methane are obtained.

The yield is 95.3% of theory, based on the initial amount of tetrabromo-p-cresol.

Melting range: 310-312°C.

Analysis: Br found: 74.5%; calcd. 74.4%.

Thermal stability: Weight loss at 300 °C = 1.1%; at 325 °C = 1.6%.

EXAMPLE 2 (Preparation)

In the same manner as in Example 1 using the same starting materials and amounts thereof, however using 2-methoxyethanol (methylglycol) as reaction medium, 200 g of colorless bis(tetrabromo-4-methylphenoxy)methane are obtained.

The yield is 93% of theory, based on the initial amount of tetrabromo-p-cresol.

Melting range: 307-310 °C.

Analysis: Br found: 74.7%; calcd. 74.4%.

Thermal stability: Weight loss at 300 °C = 0.8%; at 325 °C = 2.5%.

EXAMPLE 3 (Preparation)

a) Bromination of p-cresol

A reaction vessel is charged, in this sequence, with 5,000 g of dibromomethane, 216 g of p-cresol, purest grade (2 moles), and 20 g of anhydrous aluminum chloride. At a temperature of 20 °C maintained with moderate external cooling 1,280 g of bromine (8 moles) are gradually added within two hours. An after-reaction is allowed to occur at 20 °C for 3 hours. Then the reaction mixture is heated at 60 °C and stirred 4 times with 1,500 ml of water each in order to eliminate the aluminum compound. After the aqueous phase has been separated, the remaining organic phase is mixed with 1,000 g of 1-methoxy-2-propanol, and at a bottoms temperature of 120 °C the dibromomethane is completely distilled off from the reaction mixture. Then the mixture is cooled to 60 °C.

b) Reaction with haloalkane

Another 1,600 g of 1-methoxy-2-propanol and 84 g of sodium hydroxide (2.1 moles) are added to the mixture of a) at a temperature of 60 °C. After stirring for 15 minutes a clear solution is formed to which 261 g of dibromomethane (1.5 moles) are added. The mixture is first heated at 100 °C for 1 hour and then maintained at 110 °C for 11 hours. Then, at a bottoms temperature of 120 °C the excess dibromomethane is distilled off, and the reaction mixture is cooled. Upon workup in accordance with Example 1 there are obtained 813 g of slightly beige-colored bis(tetrabromo-4-methyl-phenoxy)-methane are obtained.

The yield is 94.5% of theory, based on the initial amount of the p-cresol.
Melting range: 312-315 °C.
Analysis: Br found: 74.4%; calcd. 74.4%.
Thermal stability: Weight loss at 300 °C = 1.0%; at 325 °C = 2.0%.

EXAMPLE 4 (Preparation)

In the same manner as in Example 3 for the bromination of p-cresol first 2,500 g of dibromomethane, 108 g of p-cresol, purest grade (1 moles), and 10 g of anhydrous aluminum chloride are charged. At a temperature of 20 °C 640 g of bromine (4 moles) are gradually added within two hours. The mixture is worked up as in Example 3, so that a solution of tetrabromo-p-cresol in 1-methoxy-2-propanol remains.

After all of the dibromomethane has been distilled off from the reaction mixture, the latter is cooled to 70 °C, and another 800 g of 1-methoxy-2-propanol and 42 g of sodium hydroxide (1.05 moles) are added. Once a clear solution has been formed, 67 g of 1,1,1-trichloroethane (0.5 moles) are added. The whole batch is heated at 100 °C, and after 1 hour the temperature is increased to 120 °C within 11 hours. Thereafter any excess 1,1,1-trichloroethane is distilled off, and the reaction mixture is cooled to 20 °C. Following the workup procedure in accordance with the preceding examples, 335 g of 1,1,1-tris(tetrabromo-4-methylphenoxy)-ethane of a bright grey color are obtained.

The yield is 77.5% of theory, based on the initial amount of the p-cresol.
Melting range: 305-306 °C.
Analysis: Br found: 74.0%; calcd. 74.1%
Thermal stability: Weight loss at 300 °C = 1.0%; at 325 °C = 2.0%.

EXAMPLE 5 (Preparation)

Upon repetition of Example 4 with recycling the mother liquor obtained herefrom of the 2nd process step (reaction with 1,1,1-trichloroethane) 420 g of 1,1,1-tris-(tetrabromo-4-methylphenoxy)ethane are obtained. The yield obtained with use of the mother liquor is 97.2% of theory. A quality deterioration does not occur thereby.

Melting range: 308-310 °C.
Analysis: Br found: 74.0%; calcd. 74.1%.
Thermal stability: Weight loss at 300 °C = 0.7%; at 325 °C = 1.6%.

EXAMPLE 6 (Preparation)

In the same manner as in Example 4, 108 g of p-cresol in 2,500 g of dibromomethane and 10 g of aluminium chloride are reacted with 640 g of bromine. The solution of tetrabromo-p-cresol obtained in this first process step is admixed with 800 g of 1-methoxy-2-propanol and 42 g of sodium hydroxide are added. Once a clear solution has been formed, 58 g of tetrachloromethane (0.374 moles) are added. The whole batch is heated at 100 °C, and after 1 hour the temperature is increased to 120 °C within 11 hours. Thereafter any excess tetrachloromethane is distilled off, and the reaction mixture is cooled to 20 °C. Following the workup procedure in accordance with the preceding examples, 252 g of nearly colorless tetrakis(tetrabromo-4-methylphenoxy)-methane are obtained.

The yield is 59.1% of theory, based on the initial amount of the p-cresol.
Melting range: 296-299 °C.
Analysis: Br found: 73.7%; calcd. 75.1%.
Thermal stability: Weight loss at 300 °C = 1.2%; at 325 °C = 1.7%.

EXAMPLE 7 (Preparation)

Upon repetition of Example 6 with recycling the mother liquor obtained herefrom of the 2nd process step (reaction with tetrachloromethane) 496 g of bright beige colored tetrakis(tetrabromo-4-methylphenoxy)-methane are obtained. The yield obtained with use of the mother liquor conforms to 116.4% of theory. Thus, the average of the yields obtained in the Examples 6 and 7 is 87.8%.
Melting range: 303-306 °C.
Analysis: Br found: 73.8%; calcd. 75.1%.
Thermal stability: Weight loss at 300 °C = 1.7%; at 325 °C = 2.6%.

EXAMPLE 8 (Preparation)

In the same manner as in Examples 3 and 4, 108 g of p-cresol, purest grade (1 mole) in 2,500 g of dibromomethane are brominated with 640 g of bromine (4 moles) in the presence of 10 g of aluminium chloride at a temperature of 20 °C. The workup is effected in accordance with said Examples so that a solution of 1 mole of tetrabromo-p-cresol in 1-methoxy-2-propanol remains.

After all of the dibromomethane has been distilled off from the reaction mixture, the latter is cooled to a temperature of 70 °C, and another 900 g of 1-methoxy-2-propanol and 42 g of sodium hydroxide (1.05 moles) are added. After a clear solution has been formed, 62 g of 1,1,2,2-tetrachloroethene (0.375 moles) are added. The reaction mixture is heated to 100 °C, and within a period of 11 hours the temperature is increased to 120 °C. Then excessive 1,1,2,2-tetrachloroethene is distilled off, the mixture is cooled to 20 °C, and the precipitated solid product is filtered off by suction. Then the filter cake is washed with 100 g of 1-methoxy-2-propanol, digested with 1 liter of water, once more washed with one liter of water and, again filtered off by suction and dried in a fresh-air drying oven at a temperature of 80 °C for 3 hours; 378 g of brightly beige-colored 1,1,2,2-tetrakis(tetrabromo-4-methyl-phenoxy)-ethene are obtained.

The yield is 88.1% of theory, based on the initial amount of tetrabromo-p-cresol.
Melting range: 309-311 °C.
Analysis: Br found: 74.2%; calcd. 74.6%.
Thermal stability: Weight loss at 300 °C = 0.6%; at 325 °C = 1.6%.

EXAMPLE 9 (Use)

Pre-dried polybutylene terephthalate (850 g.) containing 30% of glass fiber filler (PBTP-GF 30) is homogeneously mixed in a fluid mixer with 110 g of bis(tetrabromo-4-methylphenoxy)-methane (of Example 1) and 40 g of antimony trioxide. The mixture is extruded at 260 °C from a single-screw laboratory extruder and granulated. The obtained granules are molded into standard test specimens at 240 °C to 250 °C by means of an injection molding machine.

EXAMPLE 10 (Use)

In the same manner as in Example 9, 850 g of PBTB-GF 30 and 110 g of tris(tetrabromo-4-methylphenoxy)-ethane (of Example 4) are homogeneously mixed with 40 g of antimony trioxide, extruded, grandulated and injection-molded to form standard test specimens.

EXAMPLE 11 (Use)

In the same manner as in Example 9, 850 g of PBTB-GF 30 and 110 g of tetrakis(tetrabromo-4-methylphenoxy)methane (of Example 6) are homogeneously mixed with 40 g of antimony trioxide, extruded, granulated and injection-molded to form standard test specimens.

EXAMPLE 12 (Use / Comparison)

In the same manner as in Example 9, 850 g of PBTB-GF 30 and 110 g of decabromodiphenyl oxide are homogeneously mixed with 40 g of antimony trioxide, extruded, granulated and injection-molded to form standard test specimens.

EXAMPLE 13 (Use)

Pre-dried acrylonitrile/butadiene/styrene-copolymer (ABS) (780 g.)are homogeneously mixed in a fluid mixer with 170 g of bis(tetrabromo-4-methylphenoxy)-methane (of Example 1) and 50 g of antimony trioxide. The mixture is extruded at 225 °C from a single-screw laboratory extruder and granulated. The obtained granules are molded into standard test specimens at 240 °C by means of an injection molding machine.

EXAMPLE 14 (Use)

In the same manner as in Example 13, 780 g of ABS and 170 g of tris(tetrabromo-4-methylphenoxy)-ethane (of Example 4) are homogeneously mixed with 50 g of antimony trioxide, extruded, granulated and injection-molded to form standard test specimens.

EXAMPLE 15 (Use)

In the same manner as in Example 13, 780 g of ABS and 170 g of tetrakis(tetrabromo-4-methylphenoxy)-methane (of Example 6) are homogeneously mixed with 50 g of antimony trioxide, extruded, granulated and injection-molded to form standard test specimens.

EXAMPLE 16 (Use / Comparison)

In the same manner as in Example 13, 780 g of ABS and 160 g of octabromodiphenyl oxide are homogeneously mixed with 60 g of antimony trioxide, extruded, granulated and injection-molded to form standard test specimens.

The standard test specimens obtained in accordance with the Examples 9 to 16 upon climatization at 23°C/65% r.h. are subjected to the following tests:

1. Fire behavior according to Underwriters' Laboratories UL 94.
2. Tensile strength at break according to DIN 53455.
3. Elongation at break according to DIN 53455.
4. Impact resistance according to DIN 53453.
5. Migration:

Examples 9 to 12: at 140°C/100 hours.
Examples 13 to 16: at 80°C/500 hours.

6. UV test (Sun Tester)

The test results are set forth in the following Tables.

TABLE 1

| Material Composition | Neat PBTP-GF 30 | EXAMPLE 9 | 10 | 11 | 12 |
|---|---|---|---|---|---|
| Fire Test UL 94 1.6 mm | HB | VO | VO | VO | VO |
| Tensile strength at break DIN 53455 $N/mm^2$ | 110 | 110 | 107 | 111 | 105 |
| Elongation at break DIN 53455 m/m | 0.022 | 0.021 | 0.019 | 0.020 | 0.020 |
| Impact Strength DIN 53453 $kJ/m^2$ | 27.0 | 25.5 | 24.5 | 25.0 | 23.0 |
| Migration Weight loss % | - | 0.35 | 0.15 | 0.10 | 2.8 |
| Coating upon migration | none | none | none | none | yes |
| UV Sun Test | pos. | pos. | pos. | pos. | neg. |

TABLE 2

| Material Composition | Neat ABS | EXAMPLE 13 | 14 | 15 | 16 |
|---|---|---|---|---|---|
| Fire Test UL 94 1.6 mm | HB | VO | VO | VO | VO |
| Tensile strength at break DIN 53455 $N/mm^2$ | 36 | 39 | 39 | 39 | 37 |
| Elongation at break DIN 53455 m/m | 0.19 | 0.11 | 0.13 | 0.13 | 0.15 |
| Impact Strength DIN 53453 $kJ/m^2$ | No Break | 20 | 22 | 20 | 50 |
| Migration Weight loss % | - | 0 | 0 | 0 | 0 |
| Coating upon migration | none | none | none | none | none |
| UV Sun Test | pos. | pos. | pos. | pos. | neg. |

EXAMPLE 17

Preparation of bis(2,6-dibromo-4-methylphenoxy)-methane from p-cresol and bromine

a) Bromination of p-cresol to form 2,6-dibromo-p-cresol

The reaction apparatus comprises a four-neck flask, nominal contents 1 liter, equipped with a stirrer, a reflux condenser on its top, a dropping funnel and a thermometer. The top outlet of the reflux condenser was connected to an adsorption flask containing water.

In the reaction vessel 540 g of p-cresol, purest grade (5 moles) are weighed in and melted by heating at 50 °C. Within 2 hours 1630 g of bromine (10.2 moles) are added, while the temperature is maintained at between 50 °C and 60 °C by mild heating. Upon completion of the bromine addition, the mixture is allowed to after-react for another hour within the same temperature range.

An observed absorption of 786 g of hydrogen bromide in the adsorption flask corresponds to 97 % of the theoretical amount.

Subsequently to the post-reactive period as above the mixture is degassed by applying vacuum while maintaining the temperature of the reaction mixture at from 60 °C to 65 °C. To the end of the degassing operation the pressure is lowered to 20 mbar.

When allowed to cool, the reaction product solidifies to give a crystal mass of slightly violet color; 1329 g of product are obtained, representing a yield 99.9 % in based on 2,6-dibromo-p-cresol. The bromine content as determined by analysis is 60.1% which conforms to the bromine content calculated for 2,6-dibromo-p-cresol. The substance has a melting range of 45-49 °C.

b) Reaction of 2,6-dibromo-p-cresol to form bis(2,6-dibromo-4-methylphenoxy)-methane

The reaction apparatus comprises a four-neck flask, nominal contents 4 l, equipped with a stirrer, a reflux condenser on its top, a dropping funnel and a thermometer. A reflux divider is inserted between the flask and the reflux condenser and initially is adjusted to effect total reflux.

The flask is charged with, in this sequence, 2,500 g of 1-methoxy-2-propanol as the reaction medium, 532 g of 2,6-dibromo-p-cresol (2 moles) obtained in the abovedescribed process step a) and 84 g of solid sodium hydroxide (2.1 moles). After 15 minutes of stirring, the reaction mixture is homogeneous; 261 g of dibromomethane (1.5 moles) are added, and then the reaction mixture is heated at 100 °C for 1 hour and maintained at 110 °C for another 11 hours. Then the reflux divider is adjusted to allow a total withdrawal to occur, and the excess of dibromomethane is distilled off, to which purpose the bottoms temperature has to be raised to 120 °C. The reaction mixture is cooled to about 20 °C, and the precipitated solid product is filtered off by suction. The filter cake is covered with 200 g of 1-methoxy-2-propanol and again filtered by suction until dry. Then the filter cake is suspended in 3 l of water and digested for 2 hours. The solid material is once more filtered off by suction, the filter cake is washed with 2 l of water, and the water is again removed by suction filtration. The filter cake is dried in a forced-air drying oven at a temperature of 80 °C for 5 hours;
438 g of a slightly beige-colored product are obtained the bromine content of which as anlytically determined is 58.7% which almost exactly corresponds to the bromine content of 58.8% as calculated for bis(2,6-dibromo-4-methyl-phenoxy)methane. The melting range of the product is 121-124 °C.

The yield in the 2nd process step b) is 80.5% of the amount as theoretically obtainable of bis(2,6-dibromo-4-methyl-phenoxy)-methane.

**Claims**

1. Bis-or poly-(bromo-4-methylphenoxy)-alkanes and -alkenes having of the formula

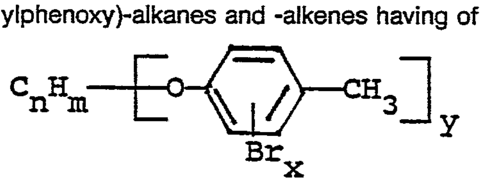

wherein
n is an integer of from 1 to 6,
m is 2 (n + z) -y,
x is an integer of from 1 to 4,
y is an integer of from 2 to 4 and
z is 0 or 1.

2. A process for preparing bis-or poly-(bromo-4-methylphenoxy)-alkanes and -alkenes of the formula

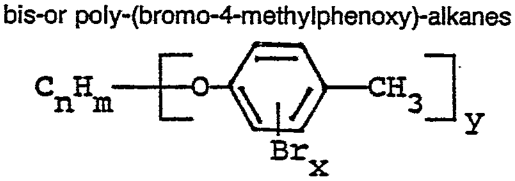

wherein
n is an integer of from 1 to 6,
m is 2 (n + z) -y,
x is an integer of from 1 to 4,
y is an integer of from 2 to 4 and
z is 0 or 1,

comprising the step of reacting alkali salts of nucleus-brominated p-cresols of the formula

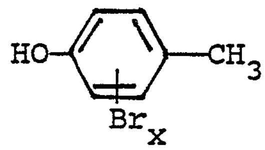

wherein x is as defined above, with bis-or poly-halo-alkanes or -alkenes of the formula

$C_nH_mHal_y$

wherein

Hal represents chlorine, bromine or iodine and n, m and y are as defined above.

3. The process according to claim 2, wherein the reaction is carried out in a glycol ether as solvent.

4. A UV-stable, low-migrating flame retardant thermoplastic synthetic molding composition comprising one or more bis-or poly-(bromo-4-methylphenoxy)-alkane or -alkene, as claimed in claim 1.

5. A thermoplastic synthetic molding composition, as claimed in claim 4, wherein the thermoplastic synthetic molding composition comprises polybutylene terephthalate or acrylonitrile-butadiene-copolymer.

6. A thermoplastic synthetic molding composition, as claimed in claim 4, wherein the bis-or poly-(bromo-4-methylphenoxy)-alkane and/or -alkene is present in an amount of from 1 to 25% by weight and wherein the composition additionally comprises from 2 to 10% by weight of antimony trioxide.

European Patent Office

# EUROPEAN SEARCH REPORT

Application number: EP 87102662.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 223 169 (HENRY J. BARDA)<br>* Claim 1; column 1; column 3, lines 35-45 *<br>-- | 1,2,4-6 | C 07 C 43/225<br>C 08 K 5/06 |
| A | US - A - 4 058 501 (ARNOLD L. ANDERSON)<br>* Abstract *<br>-- | 1,4 | |
| A | EP - A1 - 0 017 711 (RIEDEL-DE HAEN)<br>* Abstract *<br>-- | 1,2,4 | |
| A | DE - B2 - 2 659 537 (CHEMISCHE FABRIK KALK)<br>* Claims 1,3; example 3 *<br>-- | 1,2,4-6 | **TECHNICAL FIELDS SEARCHED (Int. Cl 4)** |
| A | DE - B2 - 2 112 005 (DAI-ICHI KOGYO SEIYAKU)<br>* Claim 1 *<br>-- | 1,4 | C 07 C 43/00<br>C 07 C 41/00<br>C 08 K |
| A | CH - A5 - 569 042 (INVENTA AG)<br>* Claim 1 *<br>-- | 1,4 | |
| A | DE - C1 - 3 120 556 (CHEMISCHE FABRIK KALK)<br>* Column 1, lines 3-26 *<br>---- | 1,2,4 | |

The present search report has been drawn up for all claims

| Place of search | Date of completion of the search | Examiner |
|---|---|---|
| VIENNA | 05-05-1987 | REIF |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document